(19) Europäisches Patentamt / European Patent Office / Office européen des brevets

(11) **EP 4 495 377 B1**

(12) **EUROPEAN PATENT SPECIFICATION**

(45) Date of publication and mention
of the grant of the patent:
**01.04.2026 Bulletin 2026/14**

(21) Application number: **24188822.1**

(22) Date of filing: **16.07.2024**

(51) International Patent Classification (IPC):
**E21D 11/10** $^{(2006.01)}$       **G01N 33/38** $^{(2006.01)}$

(52) Cooperative Patent Classification (CPC):
**E21D 11/102; G01N 3/10; G01N 33/383**

(54) **APPARATUS FOR DETECTING THE CONSISTENCY OF A CONCRETE CASTING, SENSORIZED PROCESSING ASSEMBLY AND RELATED DETECTION METHODS**

GERÄT ZUR FESTSTELLUNG DER KONSISTENZ EINES BETONGUSSES, SENSORISCHE VERARBEITUNGSANORDNUNG UND ENTSPRECHENDE FESTSTELLUNGSVERFAHREN

APPAREIL POUR DÉTECTER LA CONSISTANCE D'UNE COULÉE DE BÉTON, ENSEMBLE DE TRAITEMENT SENSORIEL ET MÉTHODES DE DÉTECTION CORRESPONDANTES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **17.07.2023 IT 202300014898**

(43) Date of publication of application:
**22.01.2025 Bulletin 2025/04**

(73) Proprietor: **Hinfra S.r.l.**
**15033 Casale Monferrato (AL) (IT)**

(72) Inventors:
• **GUANZIROLI, Francesco**
**22036 ERBA (Como) (IT)**
• **GUANZIROLI, Stefano**
**22036 ERBA (Como) (IT)**

(74) Representative: **Fioravanti, Corrado et al**
**Jacobacci & Partners S.p.A.**
**Corso Emilia 8**
**10152 Torino (IT)**

(56) References cited:
**AT-B- 320 313**      **JP-A- H0 270 899**
**JP-A- H08 135 386**   **JP-A- S 614 908**

EP 4 495 377 B1

## Description

Technical field

The present invention is in the field of construction for the measurement of the consistency of a concrete casting contained within a formwork. In particular, it refers to a formwork for tunnel crowns and shoulders that are under construction.

Prior art

Systems and methodologies used in the machining of a profile of a tunnel that is under construction are known in the art, i.e. systems and methods for lining tunnels or for the replacement or repair of said lining.

Typically, reinforcements are used for the construction or maintenance of the tunnel lining which are mounted inside the tunnel, in proximity to the crown and shoulders, and that are configured in such a way as to contain a concrete casting arranged so as to line the crown and shoulders. When the lining concrete has completely solidified, the reinforcement of the tunnel is removed.

The reinforcement of tunnels may be performed along the entire length of the tunnel in such a way as to perform only one machining of the entire inner surface of the tunnel. However, this methodology requires a large amount of material and considerable time to reinforce and, subsequently, remove the reinforcement from the crown and shoulders.

Alternatively, patent publications 102022000003116 and PCT/IB2023/051394 disclose tunnel lining methods that provide for the reinforcement of subsequent and adjacent tunnel segments, employing a work carriage that is movable along the axial direction of said tunnel, which allow the amount of material necessary for the reinforcement of the tunnel to be reduced, thus also accelerating construction or maintenance operations of said tunnel.

Also when using movable work carriages, before being able to proceed with removing the reinforcement of the tunnel segment whereupon work is being performed and with the reinforcement and machining of the next tunnel segment, it is necessary to wait for a period of time as defined by the relevant regulations in order to ensure the complete drying of the concrete of the current segment.

The period of time required for complete drying may, therefore, be a limiting factor in construction or maintenance processes. Furthermore, the drying time may vary depending upon many factors, for example, depending upon the concrete compound used or the casting process for the concrete casting within the formwork. Concrete compounds are in fact known that comprise additives that are intended to reduce the curing time of said concrete.

Related systems and methodologies of the prior art are disclosed in JPS614908 A, JPH0270899 A, JPH08135386 A and AT320313 B.

Summary of the invention

The object of the present invention is to optimize the formwork removal times following the curing of the concrete casting.

The aforesaid and other objects and advantages, which will be better understood from the following disclosure, are achieved according to a measurement apparatus according to one aspect of the present invention having the features set out in independent claim 1, according to a sensorized machining assembly according to claim 4, according to a measurement method according to claim 6 and a measurement method according to claim 7. Preferential embodiments of the invention are defined in the dependent claims.

A first aspect of the invention is based upon a measurement apparatus configured to measure at least one quantity representative of the consistency of a concrete casting contained within a formwork, wherein said measurement apparatus comprises:

- a penetration element defining a longitudinal axis, which is axially translatable and has a distal end portion with a distal surface configured to come into contact against and penetrate the concrete casting within the formwork,
- first actuating means designed to move the penetration element in an axial direction forward towards and away from the concrete casting,
- a formwork designed to contain the concrete casting and comprising a vertical front wall that contains the concrete casting at the front and that has at least one opening in a shape corresponding to the penetration element such as to allow it to pass from the outside to the concrete casting within the formwork;
- a support and guiding structure whereto the first actuating means and the penetration element are mounted, which are kept in positions spaced apart so as to act along said longitudinal axis, wherein said support and guiding structure is configured to rest against the front wall, and
- sensor means interposed between the penetration element and the first actuating means and arranged to provide a signal that is indicative of an axial load acting on the interface between the distal surface of the penetration element and the penetrated concrete casting, where said signal is indicative of the consistency of the concrete casting.

A second aspect of the invention is based upon a sensorized machining assembly for a crown and shoulders of a tunnel that is under construction defining an axial direction and comprising:

- at least one measurement apparatus according to the first aspect of the invention,

wherein the formwork also comprises a construction wall extending along the axial direction and adapted to form a section of the intrados of the tunnel crown and shoulders, and
wherein the front wall is adjacent to the construction wall and forms the formwork therewith,

- a frame that is movable along the axial direction and having an intrados and an extrados, said movable frame being coupled with the construction wall and the front wall and comprising third actuating means arranged on the intrados and configured to extract the construction wall along the axial direction and fourth actuating means arranged on the extrados and configured to extract the front wall along the axial direction.

A third aspect of the invention is based upon a method for measuring the consistency of a concrete casting within a formwork comprising the steps of:

a) arranging a construction wall extending along an axial direction and arranging at least one measurement apparatus according to the first aspect of the invention so that the front wall is adjacent to the construction wall and forms a formwork therewith;
b) arranging a concrete casting within the formwork such that the distal surface of the penetration element comes into contact with the concrete within the formwork;
c) actuating the first actuating means of the measurement apparatus to move the penetration element forwards towards the concrete casting by a predetermined amount such that the distal surface of the penetration element penetrates the concrete casting within the formwork;
d) by means of the sensor means of the measurement apparatus, measuring a signal that is indicative of an axial load acting on the interface between the distal surface of the penetration element and the penetrated concrete casting; and
e) calculating the peak value of the signal that is indicative of the axial load acting on the interface.

A fourth aspect of the invention is based upon a method for measuring the consistency of a concrete casting within a formwork for a crown and shoulders of a tunnel that is under construction comprising the steps of:

a) arranging a sensorized machining assembly for a tunnel crown and shoulders according to the second aspect of the present invention in a crown and shoulders for a tunnel that is under construction;
b) arranging a concrete casting within the formwork such that the distal surface of the penetration element comes into contact with the concrete within the formwork;
c) actuating the first actuating means of the measurement apparatus to move the penetration element forwards towards the concrete casting by a predetermined amount such that the distal surface of the penetration element penetrates the concrete casting within the formwork;
d) by means of the sensor means of the measurement apparatus, measuring an identification signal of an axial load acting on the interface between the distal surface of the penetration element and the penetrated concrete casting; and
e) calculating the peak value of the signal that is indicative of the axial load acting on the interface.

Brief description of the drawings

The features and advantages of the invention will emerge from the detailed description of some exemplary embodiments made with reference to the accompanying drawings, given as an indication and not intended to be limiting, wherein:

Fig. 1 is a cut-away perspective view of a measurement apparatus according to the present invention;
Fig. 2 is a sectional side view of the measurement apparatus of Fig. 1;
Fig. 3-8 are a top and sectional view of the measurement apparatus of Fig. 1 and illustrate the operation thereof; and
Fig. 9-12 are a perspective view of a sensorized machining assembly according to the present invention and illustrate the relative operation thereof.

Detailed description

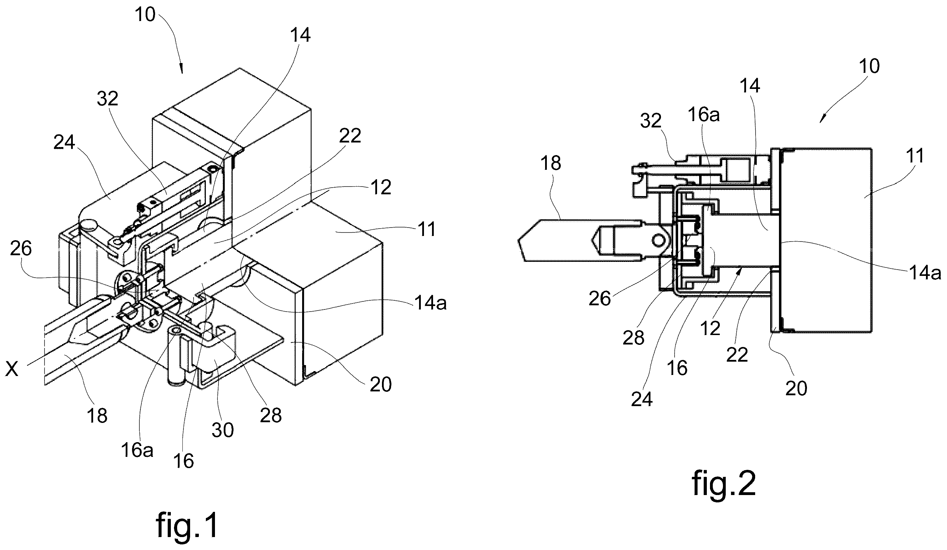

Referring to Fig. 1 to 8, a measurement apparatus 10 is configured to measure at least one quantity representative of the consistency of a casting or concrete mass contained within a formwork. For example, a formwork for crowns and shoulders of tunnels that are under construction.
The measurement apparatus 10 comprises a penetration element 12, first actuating means 18 arranged to move the penetration element 12, a formwork having a vertical front wall 20 for frontally containing the concrete casting 11 contained within the formwork, a support and guiding structure 24, and sensor means 26 interposed between the penetration element 12 and the first actuating means 18 (Fig. 1).
The penetration element 12 defines a longitudinal axis x and is translatable along said axis x in such a way as to move forwards towards the concrete casting 11 and/or away from the concrete casting 11.
The penetration element 12 has a distal end portion 14 having a distal surface 14a configured to come into contact against and to penetrate the concrete casting

11 within the formwork (Fig. 2).

Opposite the distal end portion 14, the penetration element 12 may have a proximal end portion 16 having a proximal head 16a extending radially by a greater amount than the relative distal end portion 14 (Fig. 2). In other words, the penetration element 12 may have two portions, a distal end portion 14 and a proximal end portion 16, having two different diameters.

The shape of the penetration element 12 is not limiting for the present invention. The penetration element 12 may have, for example, an overall cylindrical shape, or it may have a substantially parallelepiped shape with a rectangular or square base extended in the axial direction.

The shape of the distal surface 14a is also not limiting for the present invention. The distal surface 14a may, for example, be flat, i.e., perpendicular to the longitudinal axis x of the penetration element 12, as shown in Fig. 2.

In other embodiments that are not shown in the figures, the distal surface 14a of the penetration element 12 may, for example, be inclined with respect to the longitudinal axis x, or it may be curved, a portion of a sphere, or else a pyramid.

The penetration element 12 is moved in an axial direction forward towards and away from the concrete casting 11 by means of the first actuating means 18. In other words, the first actuating means 18 are arranged to push or retract the penetration element 12 towards or away from the concrete, respectively.

Preferably, the first actuating means 18 may be arranged axially aligned with the penetration element 12 in such a way as to facilitate the pushing thereof toward the concrete, reducing the torque generated during the pushing or retracting step of the penetration element 12. The first actuating means 18 may, for example, be a hydraulic linear actuator or an electromechanical linear actuator.

In one embodiment, the first actuating means 18 may be remotely controlled by means of a control unit (not shown in the figures). Preferably, the control unit actuates the first actuating means 18 automatically, i.e., without manual intervention on the part of an operator, on the basis of the quantity representative of the consistency of the concrete casting 11 as determined by the measurement apparatus 10.

The formwork of the measurement apparatus 10 is configured so as to contain the concrete casting 11 and comprises a front wall 20 having at least one through opening 22 shaped in a way corresponding to the penetration element 12 in such a way as to allow the passage thereof from the outside towards the concrete casting 11 within the formwork, in other words from outside the formwork towards the inside of the formwork.

Preferably, the front or containment wall 20 may be flat and may be arranged perpendicular to the longitudinal direction x, while the shape of the at least one through opening 22 is not limiting for the present invention, but may be, for example, circular, square, rectangular according to the shape of the cross section of the penetration element 12.

The measurement apparatus 10 also comprises the support and guiding structure 24, or static frame, whereto the first actuating means 18 and the penetration element 12 are mounted, which are kept in positions spaced apart so as to act along the longitudinal axis x. In other words, the first actuating means 18 and the penetration element 12, actuated by the first actuating means 18, are supported and guided in the axial movement thereof by means of the support and guiding structure 24.

The support and guiding structure 24 is configured to rest against the front wall 20 (Fig. 2). In particular, the support and guiding structure 24 may rest against a face of the front wall 20 facing away from the concrete casting 11.

The measurement apparatus 10 also comprises the sensor means 26 that are interposed between the penetration element 12 and the first actuating means 18 (Fig. 1) and that are arranged to provide a signal that is indicative of an axial load acting on the interface between the distal surface 14a of the penetration element 12 and the penetrated concrete casting 11, wherein the signal is indicative of the consistency of the concrete casting 11.

Preferably, the sensor means 26 are configured to measure at least one quantity representative of the consistency of the concrete casting 11 contained within the formwork, i.e., a quantity representative of the axial load acting on the interface between the penetration element 12 and the concrete casting 11, and to provide a signal representative of such quantity.

The sensor means 26 may, for example, be a load cell.

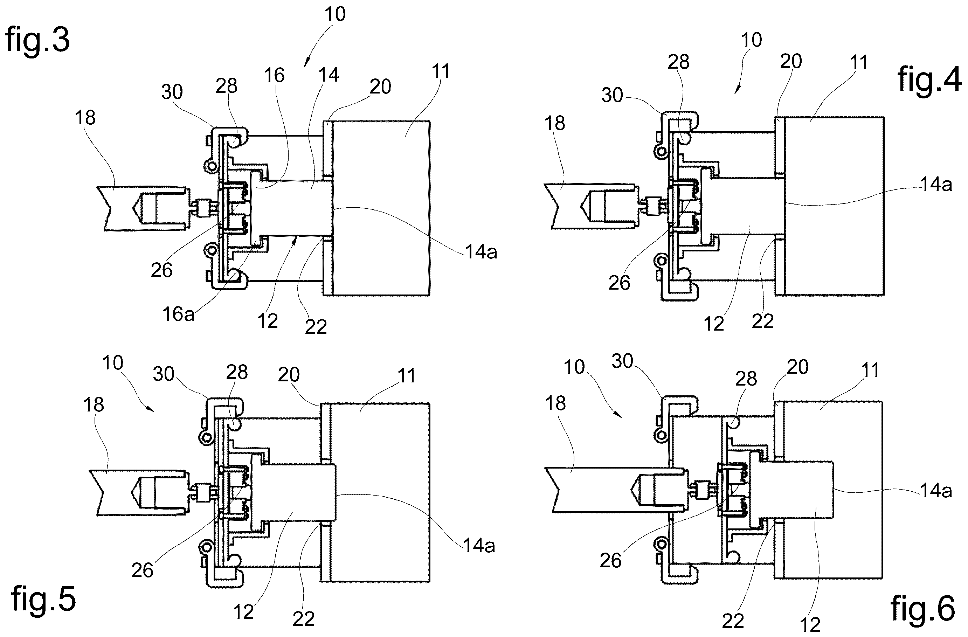

The measurement apparatus 10 may further comprise at least one gripping element 28 integral with the penetration element 12 (Fig. 3).

The gripping element 28 may be connected to the penetration element 12 by means of an intermediate element. In the embodiment shown in Fig. 3, the intermediate connection element is an S-shaped bracket so as to engage the proximal head 16a of the penetration element 12 on one side and to be connected to a face of the gripping element 28, facing the front wall 20, on the other side.

Preferably, the gripping element 28 is in the form of a plate oriented perpendicularly to the axial direction (Fig. 3). In order to facilitate gripping, the plate may have a greater thickness at the distal edge than the thickness of the central part.

In an alternative embodiment that is not shown in the figures, the measurement apparatus 10 may have a plurality of gripping elements integral with the penetration element 12 and extending therefrom in a radial direction.

In a further embodiment, the proximal head 16a of the penetration element 12, extending radially by a greater amount than the respective distal end portion, may itself act as a gripping element 28.

The gripping element 28, integral with the penetration element 12, may also be constrained to the first actuating means 18 such that they may move the gripping element 28 and/or the penetration element 12 away from the concrete casting 11 when necessary.

The measurement apparatus 10 may comprise at least one coupling element 30 fastened to the support and guiding structure 24 (Fig. 3) and configured to move between two operating positions, a first operating position, wherein the at least one coupling element 30 is engaged with and retains the at least one gripping element 28 (Fig. 3), and a second operating position, wherein the at least one coupling element 30 is disengaged from the gripping element 28 (Fig. 4).

The measurement apparatus 10 preferably comprises two coupling elements arranged laterally upon the support and guiding structure 24 and arranged to engage and disengage with the at least one gripping element 28 integral with the penetration element 12.

In the embodiment shown in Fig. 3, two coupling elements 30 may be in the shape of a C and may be arranged to engage with and retain the thicker outer edge of the gripping element.

The at least one coupling element 30 may be actuated by second actuating means 32 (Fig. 2), which may be comprised within the measurement apparatus 10 and which may be arranged so as to actuate the coupling element 30 in such a way that it is able to reach the first operating position or the second operating position.

The second actuating means 32 are preferably arranged upon an outer wall of the support and guiding structure 24, as illustrated in Fig. 2. The second actuating means 32 may, for example, be a hydraulic linear actuator or an electromechanical linear actuator.

In one embodiment, the second sensor means 26 may be controlled remotely and automatically by means of the control unit.

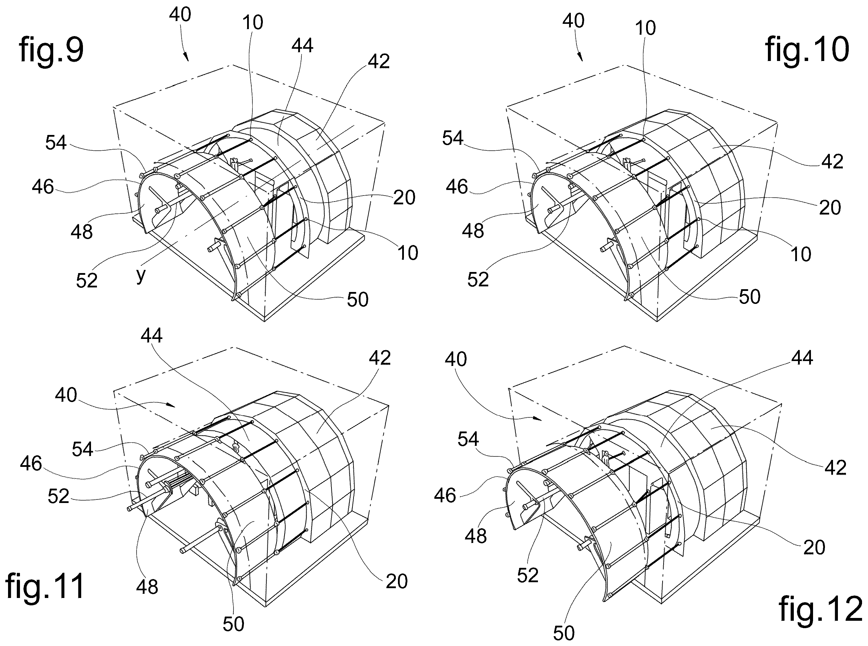

The present invention also describes a sensorized machining assembly 40 (Fig. 9-12) for a crown and shoulders of a tunnel 42 that is under construction and which defines an axial direction y. The sensorized machining assembly 40 comprises at least one measurement apparatus 10 in any of the previously described embodiments, wherein the formwork of the measurement apparatus 10 also comprises a construction or support wall 44 which extends along the axial direction y and is configured to form a section of the intrados of the tunnel crown and shoulders 42.

As illustrated in the embodiment of Fig. 9, the construction wall 44 may be an arched or semi-cylindrical wall. The shape of the construction wall 44 is not however intended to be limiting for the purposes of the invention; the construction wall 44 may in fact also be flat when tunnel crowns and shoulders with an intrados having substantially flat surfaces are made.

The construction wall 44 is preferably movable, for example, it may be moved by means of wheels that engage in rails or by means of rubber wheels.

In the exemplary embodiment shown in Fig. 9, a sensorized machining assembly 40 is shown having three measurement apparatuses 10, wherein only two thereof are visible in the figure.

In the sensorized machining assembly 40, the front wall 20 is adjacent to the construction wall 44 (Fig. 9) and forms therewith the formwork arranged to contain a concrete casting 11 in such a way as to be able to form a tunnel segment or header.

The sensorized machining assembly 40 also comprises a movable frame 46 which is movable along the axial direction y of the crown and shoulders of a tunnel 42 that is under construction and has an intrados 48 and an extrados 50. As with the construction wall 44, the frame may also be movable, for example, it may be moved by means of wheels that engage in rails or by means of rubber wheels.

As shown in Fig. 9-12, the movable frame 46 is coupled with the construction wall 44 and the front wall 20 and comprises third actuating means 52 arranged upon the intrados 48 and configured to extract the construction wall 44 along the axial direction y, and further comprises fourth actuating means 54 arranged upon the extrados 50 and configured to extract the front wall 20 along the axial direction y. In said description, the term "extract" signifies that the third 52 and fourth 54 actuating means 54 are adapted to move the construction wall 44 and the front wall 20 away from the formed tunnel crown and shoulders 42, i.e., wherein the concrete casting has cured.

The third actuating means 52 and the fourth actuating means 54 are preferably operated remotely and automatically by the control unit.

Preferably the first actuating means 18 of the at least one measurement apparatus 10 are arranged upon the extrados 50 of the movable frame 46 and are interposed between the fourth actuating means 54.

In one embodiment, the sensorized machining assembly 40 comprises, as a function of the size of the crown and shoulders, a number of measurement apparatuses. For example, for a crown and shoulders with a width equal to 20 m, three measurement apparatuses may be envisaged, and for crowns and shoulders with larger dimensions a greater number of measurement apparatuses may be envisaged.

When a plurality of measurement apparatuses are present, they are preferably arranged in angularly spaced positions upon the extrados 50 of the movable frame 46 and interposed with the fourth actuating means 54. For example, a measurement apparatus 10 may be arranged in proximity to the key, and two other measurement apparatuses may be arranged in proximity to respective piers of the segment or header of the tunnel crown and shoulders 42 being constructed. This exemplifying arrangement is particularly advantageous insofar as it identifies significant measurement or test areas that may be able to provide data representative of the entire crown and shoulders. Other arrangements of the measurement apparatuses 10 are however possible depending upon the size of the tunnel crown and shoulders.

The present invention also describes a method for measuring the consistency of a concrete casting 11 within a formwork that comprises arranging a construction wall 44, extending along an axial direction y, and arranging at least one measurement apparatus 10, according to any of the embodiments described above, in such a way that the front wall 20 is adjacent to the construction wall 44 and forms therewith a formwork.

Preferably, a plurality of measurement apparatuses 10 is used to measure the consistency of the concrete casting 11 within the formwork, and the plurality of measurement apparatuses 10 is operated synchronously, i.e., they are operated simultaneously, by means of the control unit so that all the measurement apparatuses 10 measure a quantity representative of the consistency of a concrete casting 11 contained within the formwork at the same instant. In this embodiment, the formwork will be removed when all the measurement apparatuses 10 used measure a peak value Fn of the signal that is indicative of the axial load acting on the interface equal to or greater than a limit value Flim, a limit value necessary to allow the formwork to be removed in complete safety.

The method provides for arranging a concrete casting 11 within the formwork formed by the front wall 20 and the construction wall 44 so that the distal surface 14a of the penetration element 12 comes into contact with the concrete within the formwork (Fig. 3).

Actuating the first actuating means 18 of the measurement apparatus 10 to move the penetration element 12 forwards towards the concrete casting 11 by a predetermined amount so that the distal surface 14a of the penetration element 12 penetrates the concrete casting 11 within the formwork (Fig. 5 and 6).

By means of the sensor means 26 of the measurement apparatus 10, measuring a signal that is indicative of an axial load acting on the interface between the distal surface 14a of the penetration element 12 and the penetrated concrete casting 11.

Calculating the peak value Fn of the signal that is indicative of the axial load acting on the interface.

The present invention also describes a method for measuring the consistency of a concrete casting 11 within a formwork for a crown and shoulders of a tunnel 42 that is under construction that comprises arranging a sensorized machining assembly 40 for a tunnel crown and shoulders 42 according to any of the preceding embodiments in a crown and shoulders of a tunnel 42 that is under construction (Fig. 9).

Arranging a concrete casting 11 within the formwork (Fig. 10) in such a way that the distal surface 14a of the penetration element 12 comes into contact with the concrete within the formwork.

Actuating the first actuating means 18 of the measurement apparatus 10 in order to move the penetration element 12 forwards towards the concrete casting 11 by a predetermined amount in such a way that the distal surface 14a of the penetration element 12 penetrates the concrete casting 11 within the formwork.

By means of the sensor means 26 of the measurement apparatus 10, measuring an identification signal of an axial load acting on the interface between the distal surface 14a of the penetration element 12 and the penetrated concrete casting 11.

Calculating the peak value Fn of the signal that is indicative of the axial load acting on the interface.

Preferably, in the control methods for measuring the consistency of a concrete casting 11 within a formwork and within a formwork for a tunnel crown and shoulders 42, the steps of actuating the first actuating means 18, measuring the identification signal of an axial load acting on the interface and calculating the peak value Fn of such signal are repeated in sequence with a time interval ΔTn until the peak value Fn of the signal that is indicative of the axial load acting on the interface becomes equal to or greater than a preset limit value Flim. The limit value Flim is the value necessary in order to ensure the safe removal of the formwork and may be a function of the mechanical and/or physical and/or chemical properties of the concrete used, i.e., the rheology of the concrete. For example, the preset limit value Flim for the measurement apparatus 10 may be 5 tons.

The time interval ΔTn between successive actuations of the first actuating means 18 may be calculated using the following two steps:

- calculating the variation of the peak value ΔFn as a function of the current peak value Fn and the previous peak value Fn-1. Preferably, the variation ΔFn may be calculated as the difference between the current peak value Fn and the previous peak value Fn-1. For example, the variation ΔFn may be calculated using the formula (1)

$$\Delta F_n \ = \ F_n - F_{n-1} \qquad (1)$$

- calculating the time interval ΔTn as a function of the previous time interval ΔTn-1, of a parameter representative of the desired variation in the peak value k and the variation in the peak value ΔFn. Preferably, the time interval ΔTn may be calculated as the ratio of the product of the previous time interval ΔTn-1 and of the parameter representative of the desired variation in the peak value k with respect to the peak value ΔFn. For example, the time interval ΔTn may be calculated using the formula (2)

$$\Delta T_n \ = \ \frac{T_{n-1}*k}{\Delta F_n} \qquad (2)$$

The parameter representative of the desired variation in the peak value k may be in the range of between about 30 N and about 120 N and is a function of the type and/or rheology of the concrete used. The term "rheology" refers to the behavior, i.e. the physical-chemical properties of the concrete in the fresh state and the relative behavior in the transition from the fresh state to the hardened state. For example, the parameter value representative of the desired variation in the peak value k may be 60 N when the concrete contains a quick-setting concrete.

At the first calculation cycle of the time interval $\Delta Tn$ the previous peak value Fn-1 and the value of the previous time interval $\Delta Tn-1$ may have a value that is defined a priori, for example by an operator, based upon the mechanical and/or physical and/or chemical features of the concrete used.

The time interval $\Delta Tn$ may be variable between a minimum value x and a maximum value y depending upon the type and/or composition of concrete used for the casting.

In a preferred embodiment, the time interval $\Delta Tn$ may be in the range of between a minimum value x of about 45 s and a maximum value y of about 300 s. Such range of values for the time interval $\Delta Tn$ is particularly advantageous when using concrete that comprises a quick-setting cement and that has controlled rheology.

The time interval $\Delta Tn$ may be monitored during subsequent cycles for measuring the consistency of a concrete casting and, when the time interval $\Delta Tn$ corresponds to a minimum time value x for a predetermined number of consecutive cycles z, the method preferably also comprises the steps of:

- calculating the average value $\Delta Fm$ of the variations in the peak value $\Delta Fn$ for the predetermined number of consecutive cycles z;
- comparing the variations in the peak value $\Delta Fn$ of each of the predetermined number of consecutive cycles with the average value $\Delta Fm$; and
- if the peak values Fn of each of the predetermined number of consecutive cycles are in the range between approximately $a*\Delta Fm$ and approximately $b*\Delta Fm$, setting the time interval $\Delta Tn$ to a predetermined value h.

In an exemplary embodiment, the steps just described may be performed when the time interval $\Delta Tn$ is equal to 45 s for a number of consecutive cycles equal to three.

In an exemplary embodiment, the average value $\Delta Fm$ of the variations in the peak value $\Delta Fn$ for the predetermined number of consecutive cycles z may be calculated using the formula (3)

$$\Delta F_m = \frac{\Delta F_1 + \Delta F_2 + \cdots + \Delta F_z}{z} \qquad (3)$$

In an exemplary embodiment, the values a and b defining the range of the average value $\Delta Fm$ may be 0.9 and 1.1, respectively, and the predetermined value h, whereto the time interval $\Delta Tn$ may be reset, may be equal to 180 s.

Fig. 3-8 show the operation of an exemplary embodiment of a measurement apparatus 10 according to the present invention.

Fig. 3 shows the measurement apparatus 10 for measuring at least one quantity representative of the consistency of a concrete casting 11 within the formwork. In this example, the measurement apparatus has two coupling elements 30 which in Fig. 3 are shown in the first operating position, i.e. engaged with the gripping element 28 in such a way as to retain the penetration element 12 and wherein the sensor means 26 measure only the hydrostatic thrust of the concrete casting 11 within the formwork.

Fig. 4 shows the two coupling means 30 in the second operating position, i.e. disengaged from the gripping element 28, in such a way that the penetration element 12 is free to translate along the longitudinal direction x when actuated by the first actuating means 18.

Fig. 5 shows the actuation of the first actuating means 18 in such a way that the penetration element 12 is pushed against the concrete casting 11 and the sensor means 26 provide a signal that is indicative of an axial load acting on the interface between the distal surface 14a of the penetration element 12 and the penetrated concrete casting 11.

The actuation of the first actuating means 18 of the actuation system may occur when the concrete casting has been arranged within the formwork, i.e., when the concrete casting 11 is still liquid, this allows for a series of advantages, including acquiring a series of data relating to the hardening of the concrete and, therefore, producing a curing development curve over time, being able to observe the passage of the concrete from the fluid state to the plastic state and then to the hardened state, having greater control over the entire curing of the concrete.

The actuation of the penetration element 12 is repeated a plurality of times (Fig. 6) at intervals of time that are calculated as described above, until the peak value Fn of the signal that is indicative of the axial load acting on the interface becomes equal to or greater than a preset limit value Flim.

The interval of time between one actuation of the penetration element 12 and the next may expand, i.e., extend, when the concrete is in a more liquid state or may decrease when the concrete is in a more solid or hardened state.

For example, for each cycle, the penetration element 12 may penetrate the concrete in a range of about 0.5 mm to about 1 mm, and the maximum stroke of the penetration element 12 may be about 10 cm.

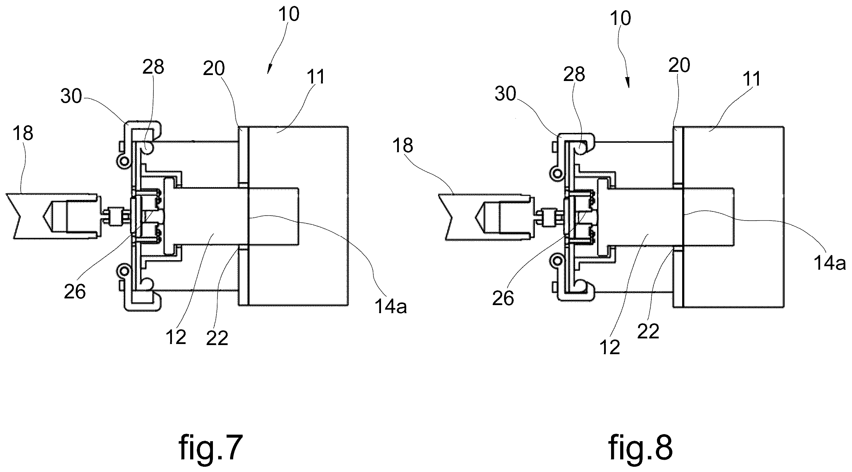

Once the axial load on the interface, as measured by the sensor means 26, becomes equal to or greater than a preset limit value Flim, the at least one penetration element 12 is automatically extracted from the concrete and returned to the initial position, as shown in Fig. 7, and two coupling elements 30 are again engaged with the gripping element 28, as shown in Fig. 8, in order to lock the penetration element 12.

Using a measurement apparatus 10, such as the one described by the present invention, advantageously allows the consistency of the concrete to be monitored over time, reducing the waiting time required in order to define the correct state of curing, removing the formwork and proceeding with the casting of a subsequent header.

Fig. 9-12 show an exemplary embodiment of a sensorized machining assembly 40 according to the present invention and the operation thereof for constructing a crown and shoulders of a tunnel 42 that is under construction.

In the example shown in Fig. 9, the sensorized machining assembly 40 comprising three measurement apparatuses, wherein only two thereof are visible in the figures, respectively arranged in the key and in the piers of the tunnel crown and shoulders. Also shown are three cylinders or actuators arranged on the intrados 48 of the movable frame 46 arranged to extract the construction wall 44 away from the formed tunnel crown and shoulders 42, nine cylinders or actuators arranged on the extrados 50 of the movable frame 46 arranged to extract the front wall 20 away from the formed tunnel crown and shoulders 42. In the configuration shown in the figures, three of the nine cylinders on the extrados 50 may have dual functions, i.e., both as a function of fourth actuating means 54 for extracting the front wall 20 and as a function of first actuating means 18 of the measurement apparatus 10. The function of such actuators may be adjusted by means of the control unit.

Fig. 10 shows how the construction wall 44 and the front wall 20 together form the formwork wherein the concrete may be deposited in such a way as to make a new tunnel crown header or segment. The degree of curing of the concrete, i.e., the consistency thereof, may be measured by means of the measurement apparatus wherein the actuators function as first actuating means 18, as has been explained above.

Fig. 11 shows how, when the degree of curing of the concrete is adequate, the actuators on the intrados 48 are actuated, for example by means of the control unit, and the construction wall 44 is extracted from the formed tunnel crown and shoulders 42, while all of the actuators on the extrados 50 act as a means of contrast for the front wall 20, in such a way as to prevent relative displacements.

Fig. 12 shows that the front wall 20 is also extracted away from the portion of the tunnel formed by the actuation of those pistons present on the extrados 50 of the mobile frame 46, all nine functioning as third actuating means 52 and controlled by the control unit.

In a preferred embodiment, the control unit actuates the first 18, second 32, third 52 and fourth 54 actuating means based upon measurements obtained from the measurement apparatus, allowing one or more measurement apparatuses 10 and/or the sensorized machining assembly 40 to be actuated automatically, i.e., without manual intervention on the part of an operator.

While specific embodiments of the invention have been described, it is to be understood that this disclosure has been provided for illustrative purposes only and that the invention is not to be limited in any way by it. Various modifications will be evident to those skilled in the sector in the light of the preceding examples. The scope of the invention is limited only by the appended claims.

## Claims

1. A measurement apparatus (10) configured to measure at least one quantity that is representative of the consistency of a concrete casting (11) contained within a formwork, wherein said measurement apparatus (10) comprises:

   - a penetration element (12) defining a longitudinal axis (x), axially translatable and having a distal end portion (14) with a distal surface (14a) configured to come in contact with and penetrate the concrete casting (11) within the formwork,
   - first actuating means (18) arranged to move the penetration element (12) in an axial direction forward towards and away from the concrete casting (11),
   - a formwork configured to contain the concrete casting (11) and comprising a vertical front wall (20) that contains at the front the concrete casting (11) and that has at least one through opening (22) shaped in a way corresponding to the penetration element (12) in order to allow it to pass from the outside towards the concrete casting (11) within the formwork;
   - a support and guiding structure (24) whereto the first actuating means (18) and the penetration element (12) are mounted, which are kept in positions spaced apart so as to act along said longitudinal axis (x), wherein said support and guiding structure (24) is configured so as to rest against the front wall (20), and
   - sensor means (26) interposed between the penetration element (12) and the first actuating means (18) and arranged to make available a signal that is indicative of an axial load acting on the interface between the distal surface (14a) of the penetration element (12) and the penetrated concrete casting (11), where said signal is indicative of the consistency of the concrete casting (11).

2. A measurement apparatus (10) according to claim 1, further comprising:

at least one gripping element (28) integral with the penetration element (12) and constrained to the first actuating means (18) to move the penetration element (12) away from the concrete casting (11),
at least one coupling element (30) fastened to the support and guiding structure (24) and configured to move between two operating positions, a first operating position wherein the at least one coupling element (30) is engaged with and retains the at least one gripping element (28), and a second operating position wherein the at least one coupling element (30) is disengaged from the at least one gripping element (28), and
second actuating means (32) arranged to actuate the at least one coupling element (30) in such a way that the at least one coupling element (30) is able to reach the first operating position or the second operating position.

3. A measurement apparatus (10) according to claim 1 or 2, wherein the penetration element (12) has a proximal end portion (16), opposite the distal end portion (14), having a proximal head (16a) radially extended by a greater amount than the distal end portion (14).

4. A sensorized machining assembly (40) for a crown and shoulders of a tunnel (42) that is under construction defining an axial direction (y), comprising:

- at least one measurement apparatus (10) according to claim 1 or 2 or 3,
wherein the formwork also comprises a construction wall (44) extending along the axial direction (y) and adapted to form a segment of the intrados of the tunnel crown and shoulders (42), and
wherein the front wall (20) is adjacent to the construction wall (44) and forms therewith a formwork arranged to contain a concrete casting (11),
- a frame (46) that is movable along the axial direction (y) and having an intrados (48) and an extrados (50), said movable frame (46) being coupled with the construction wall (44) and with the front wall (20) and comprising third actuating means (52) arranged upon the intrados (48) and configured to extract the construction wall (44) along the axial direction (y) and fourth actuating means (54) arranged upon the extrados (50) and configured to extract the front wall (20) along the axial direction (y).

5. A sensorized machining assembly (40) according to claim 4, wherein the first actuating means (18) of the at least one measurement apparatus (10) are arranged upon the extrados (50) of the movable frame (46) interposed between the fourth actuating means (54).

6. A method for measuring the consistency of a concrete casting (11) within a formwork comprising the steps of:

a) arranging a construction wall (44) extending along an axial direction (y) and arranging at least one measurement apparatus (10) according to any one of the claims from 1 to 3 in such a way that the front wall (20) is adjacent to the construction wall (44) and forms a formwork therewith;
b) arranging a concrete casting (11) within the formwork in such a way that the distal surface (14a) of the penetration element (12) comes into contact with the concrete within the formwork;
c) actuating the first actuating means (18) of the measurement apparatus (10) in order to move the penetration element (12) forwards towards the concrete casting (11) by a predetermined amount so that the distal surface (14a) of the penetration element (12) penetrates the concrete casting (11) within the formwork;
d) by means of the sensor means (26) of the measurement apparatus (10) measuring a signal that is indicative of an axial load acting on the interface between the distal surface (14a) of the penetration element (12) and the penetrated concrete casting (11); and
e) calculating a peak value ($F_n$) of the signal that is indicative of the axial load acting on the interface.

7. A method for measuring the consistency of a concrete casting (11) within a formwork for a crown and shoulders of a tunnel (42) that is under construction comprising the steps of:

a) arranging a sensorized machining assembly (40) for a tunnel crown and shoulders (42) according to claim 4 or 5 within a crown and shoulder of a tunnel (42) that is under construction;
b) arranging a concrete casting (11) within the formwork in such a way that the distal surface (14a) of the penetration element (12) comes into contact with the concrete within the formwork;
c) actuating the first actuating means (18) of the measurement apparatus (10) in order to move the penetration element (12) forwards towards the concrete casting (11) by a predetermined amount such that the distal surface (14a) of the penetration element (12) penetrates the concrete casting (11) within the formwork;

d) by means of the sensor means (26) of the measurement apparatus (10) measuring a signal indicative of an axial load acting on the interface between the distal surface (14a) of the penetration element (12) and the penetrated concrete casting (11); and

e) calculating a peak value (Fn) of the signal indicative of the axial load acting on the interface.

8. A method according to claim 6 or 7, wherein the steps c), d) and e) are repeated sequentially with a time interval ($\Delta$Tn), until the peak value (Fn) of the signal indicative of the axial load acting on the interface becomes equal to or greater than a preset limit value (Flim).

9. A method according to claim 8, wherein calculating the time interval ($\Delta$Tn) comprises the steps of:

i) calculating the variation in the peak value ($\Delta$Fn) as a function of the current peak value (Fn) and the previous peak value (Fn-1); and

j) calculating the time interval ($\Delta$Tn) as a function of the previous time interval ($\Delta$Tn-1), a parameter representing the desired variation of the peak value (k) and the variation of the peak value ($\Delta$Fn).

10. A method according to claim 8 or 9, wherein the time interval ($\Delta$Tn) is in the range between a minimum value (x) of about 45 s and a maximum value (y) of about 300 s.

11. A method according to any one of claims 8 to 10, wherein when the time interval ($\Delta$Tn) corresponds to a minimum time value (x) for a predetermined number of consecutive cycles (z), the method comprising the steps of:

m) calculating the mean value ($\Delta$Fm) of the peak value variations ($\Delta$Fn) for the predetermined number of consecutive cycles (z);

n) comparing the variations in the peak value ($\Delta$Fn) of each of the predetermined number of consecutive cycles with the mean value ($\Delta$Fm); and

o) if the peak values (Fn) of each of the predetermined number of consecutive cycles are within the range between approximately a*$\Delta$Fm and approximately b*$\Delta$Fm, setting the time interval ($\Delta$Tn) to a predetermined value (h).

**Patentansprüche**

1. Messeinrichtung (10), die dazu konfiguriert ist, mindestens eine Größe zu messen, die repräsentativ für die Konsistenz eines Betongusses (11) ist, der innerhalb einer Schalung enthalten ist, wobei die Messeinrichtung (10) umfasst:

- ein Eindringelement (12), das eine Längsachse (x) definiert, die axial verschiebbar ist und aufweisend einen distalen Endabschnitt (14) mit einer distalen Fläche (14a), die dazu konfiguriert ist, mit dem Betonguss (11) in Kontakt zu kommen und innerhalb der Schalung in diesen einzudringen,

- erste Betätigungsmittel (18), die eingerichtet sind, das Eindringelement (12) in eine axiale Richtung vorwärts zu und weg von dem Betonguss (11) zu bewegen,

- eine Schalung, die dazu konfiguriert ist, den Betonguss (11) zu enthalten, und eine vertikale Stirnwand (20) umfasst, die den Betonguss (11) vorne hält und mindestens eine Durchgangsöffnung (22) aufweist, die in einer Weise ausgebildet ist, die mit dem Eindringelement (12) übereinstimmt, um dessen Durchgang von außen zu dem Betonguss (11) innerhalb der Schalung zu ermöglichen,

- eine Trag- und Führungsstruktur (24), an der die ersten Betätigungsmittel (18) und das Eindringelement (12) eingebaut sind, die in voneinander beabstandeten Stellungen gehalten werden, um entlang der Längsachse (x) zu wirken, wobei die Trag- und Führungsstruktur (24) dazu konfiguriert ist, auf die Stirnwand (20) aufzusetzen, und

- Sensormittel (26), die zwischen dem Eindringelement (12) und den ersten Betätigungsmitteln (18) angeordnet sind und eingerichtet sind, ein Signal bereitzustellen, das für eine axiale Last bezeichnend ist, die an der Grenzfläche zwischen der distalen Fläche (14a) des Eindringelements (12) und dem eingedrungenen Betonguss (11) wirkt, wobei das Signal für die Konsistenz des Betongusses (11) bezeichnend ist.

2. Messeinrichtung (10) nach Anspruch 1, ferner umfassend:

- mindestens ein Greifelement (28), das einstückig mit dem Eindringelement (12) ausgebildet ist und an die ersten Betätigungsmittel (18) befestigt wird, um das Eindringelement (12) weg von dem Betonguss (11) zu bewegen,

- mindestens ein Kupplungselement (30), das an der Trag- und Führungsstruktur (24) befestigt wird und dazu konfiguriert ist, sich zwischen zwei Betriebsstellungen zu bewegen, einer ersten Betriebsstellung, wobei das mindestens eine Kupplungselement (30) mit dem mindestens einen Greifelement (28) in Eingriff steht und dieses hält, und einer zweiten Betriebsstellung wobei das mindestens eine Kupplungselement (30) von dem mindestens einen Greifelement (28) getrennt wird, und
- zweite Betätigungsmittel (32), die eingerichtet sind, das mindestens eine Kupplungselement (30) zu betätigen, sodass das mindestens eine Kupplungselement (30) in der Lage ist, die erste Betriebsstellung oder die zweite Betriebsstellung zu erreichen.

3. Messeinrichtung (10) nach Anspruch 1 oder 2, wobei das Eindringelement (12) einen proximalen Endabschnitt (16) aufweist, der dem distalen Endabschnitt (14) gegenüberliegt, aufweisend einen proximalen Kopf (16a), der sich radial um einen größeren Umfang als der distale Endabschnitt (14) erstreckt.

4. Sensorische Verarbeitungsanordnung (40) für einen Kopf und Schultern eines Tunnels (42), der sich im Bau befindet und eine axiale Richtung (y) definiert, umfassend:

   - mindestens eine Messeinrichtung (10) nach einem der Ansprüche 1 oder 2 oder 3,

      wobei die Schalung auch eine Bauwand (44) umfasst, die sich entlang der axialen Richtung (y) erstreckt und eingerichtet ist, ein Segment der Unterseite des Tunnelkopfes und Schultern (42) zu bilden, und wobei die Stirnwand (20) an die Bauwand (44) angrenzend ist und damit eine Schalung bildet, die eingerichtet ist, einen Betonguss (11) aufzunehmen,

   - einen Rahmen (46), der entlang der axialen Richtung (y) beweglich ist und eine Unterseite (48) und eine Außenseite (50) aufweist, wobei der bewegliche Rahmen (46) mit der Bauwand (44) und mit der Stirnwand (20) gekoppelt ist und dritte Betätigungsmittel (52) umfasst, die auf der Unterseite (48) angeordnet sind und dazu konfiguriert sind, die Bauwand (44) entlang der axialen Richtung (y) herauszuziehen, sowie vierte Betätigungsmittel (54), die auf dem Außenseite (50) angeordnet sind und dazu konfiguriert sind, die Stirnwand (20) entlang der axialen Richtung (y) herauszuziehen.

5. Sensorische Verarbeitungsanordnung (40) nach Anspruch 4, wobei die ersten Betätigungsmittel (18) der mindestens einen Messeinrichtung (10) auf der Außenseite (50) des beweglichen Rahmens (46) zwischen den vierten Betätigungsmitteln (54) angeordnet sind.

6. Verfahren zur Messung der Konsistenz eines Betongusses (11) innerhalb einer Schalung, umfassend die folgenden Schritte:

   a) Anordnen einer Bauwand (44), die sich entlang einer axialen Richtung (y) erstreckt, und Anordnen mindestens einer Messeinrichtung (10) nach einem der Ansprüche 1 bis 3, sodass die Stirnwand (20) an die Bauwand (44) angrenzend ist und damit eine Schalung bildet,
   b) Anordnen eines Betongusses (11) innerhalb der Schalung, sodass die distale Fläche (14a) des Eindringelements (12) mit dem Beton innerhalb der Schalung in Kontakt kommt,
   c) Betätigen der ersten Betätigungsmittel (18) der Messeinrichtung (10), um das Eindringelement (12) vorwärts zu dem Betonguss (11) um einen vorbestimmten Umfang zu bewegen, sodass die distale Fläche (14a) des Eindringelements (12) in den Betonguss (11) innerhalb der Schalung eindringt,
   d) Messen, mittels der Sensormittel (26) der Messeinrichtung (10), eines Signals, das für eine axiale Last bezeichnend ist, die an der Grenzstelle zwischen der distalen Fläche (14a) des Eindringelements (12) und dem eingedrungenen Betonguss (11) wirkt, und
   e) Berechnen eines Spitzenwerts (Fn) des Signals, das für die axiale Last, die an der Grenzstelle wirkt, bezeichnend ist.

7. Verfahren zur Messung der Konsistenz eines Betongusses (11) innerhalb einer Schalung für einen Kopf und Schultern eines Tunnels (42), der sich im Bau befindet, umfassend die folgenden Schritte:

   a) Anordnen einer sensorischen Verarbeitungsanordnung (40) für einen Tunnelkopf und Schultern (42) nach Anspruch 4 oder 5 innerhalb eines Kopfs und Schultern eines Tunnels (42), der sich im Bau befindet,
   b) Anordnen eines Betongusses (11) innerhalb der Schalung, sodass die distale Fläche (14a) des Eindringelements (12) mit dem Beton innerhalb der Schalung in Kontakt kommt,
   c) Betätigen der ersten Betätigungsmittel (18) der Messeinrichtung (10), um das Eindringelement (12) vorwärts um einen vorbestimmten Umfang zu dem Betonguss (11) zu bewegen, sodass die distale Fläche (14a) des Eindringelements (12) in den Betonguss (11) innerhalb der Schalung eindringt,
   d) Messen, mittels der Sensormittel (26) der Messeinrichtung (10), eines Signals, das für eine axiale Last bezeichnend ist, die an der Grenzstelle zwischen der distalen Fläche (14a) des Eindringelements (12) und dem eingedrungenen Betonguss (11) wirkt, und
   e) Berechnen eines Spitzenwerts (Fn) des Signals, das für die axiale Last, die an der Grenzstelle wirkt, bezeichnend ist.

8. Verfahren nach Anspruch 6 oder 7, wobei die Schritte c), d) und e) sequenziell in einem Zeitintervall (ΔTn) wiederholt werden, bis der Spitzenwert (Fn) des Signals, das für die axiale Last, die an der Grenzstelle wirkt, bezeichnend ist, gleich oder größer als ein voreingestellter Grenzwert (Flim) wird.

9. Verfahren nach Anspruch 8, wobei Berechnen des Zeitintervalls (ΔTn) die folgenden Schritte umfasst:

   i) Berechnen der Änderung des Spitzenwerts (ΔFn) in Abhängigkeit von dem aktuellen Spitzenwert (Fn) und von dem vorherigen Spitzenwert (Fn-1), und
   j) Berechnen des Zeitintervalls (ΔTn) in Abhängigkeit von dem vorherigen Zeitintervall (ΔTn-1), einem Parameter, der die gewünschte Änderung des Spitzenwerts (k) darstellt, und der Änderung des Spitzenwerts (ΔFn).

10. Verfahren nach Anspruch 8 oder 9, wobei das Zeitintervall (ΔTn) in einem Bereich zwischen einem Mindestwert (x) von etwa 45 s und einem Höchstwert (y) von etwa 300 s liegt.

11. Verfahren nach einem der Ansprüche 8 bis 10, wobei, wenn das Zeitintervall (ΔTn) einem Mindestzeitwert (x) für eine vorbestimmte Anzahl aufeinanderfolgender Zyklen (z) entspricht, das Verfahren die folgenden Schritte umfasst:

   m) Berechnen des Mittelwerts (ΔFm) der Änderungen der Spitzenwerte (ΔFn) für die vorbestimmte Anzahl aufeinanderfolgender Zyklen (z),
   n) Vergleichen der Änderungen des Spitzenwertes (ΔFn) jedes der vorbestimmten aufeinanderfolgenden Zyklen mit dem Mittelwert (ΔFm), und
   o) wenn die Spitzenwerte (Fn) jedes der vorbestimmten aufeinanderfolgenden Zyklen in einem Bereich zwischen etwa a*ΔFm und etwa b*ΔFm liegen, Einstellen des Zeitintervalls (ΔTn) auf einen vorbestimmten Wert (h).

**Revendications**

1. Un appareil de mesure (10) configuré pour mesurer au moins une grandeur représentative de la consistance d'un coulage de béton (11) contenu à l'intérieur d'un coffrage, dans lequel ledit appareil de mesure (10) comprend :

   un élément de pénétration (12) définissant un axe longitudinal (x), translatable axialement et présentant une partie d'extrémité distale (14) avec une surface distale (14a) configurée pour entrer en contact avec et pénétrer le coulage de béton (11) à l'intérieur du coffrage,
un premier moyen d'actionnement (18) agencé pour déplacer l'élément de pénétration (12) dans une direction axiale vers et à l'écart du coulage de béton (11),
un coffrage configuré pour contenir le coulage de béton (11) et comprenant une paroi frontale verticale (20) qui contient frontalement le coulage de béton (11) et qui présente au moins une ouverture traversante (22) façonnée de manière correspondante à l'élément de pénétration (12) afin de lui permettre de passer de l'extérieur vers le coulage de béton (11) à l'intérieur du coffrage ;
une structure de support et de guidage (24) sur laquelle le premier moyen d'actionnement (18) et l'élément de pénétration (12) sont montés, lesquels sont maintenus dans des positions espacées de manière à agir le long dudit axe longitudinal (x), dans laquelle ladite structure de support et de guidage (24) est configurée pour venir en butée contre la paroi frontale (20), et
un moyen de détection (26) interposé entre l'élément de pénétration (12) et le premier moyen d'actionnement (18) et agencé pour fournir un signal indicatif d'une charge axiale agissant sur l'interface entre la surface distale (14a) de l'élément de pénétration (12) et le coulage de béton (11) pénétré, où ledit signal est indicatif de la consistance du coulage de béton (11).

2. Un appareil de mesure (10) selon la revendication 1, comprenant en outre :

   au moins un élément de préhension (28) solidaire de l'élément de pénétration (12) et contraint au premier moyen d'actionnement (18) pour déplacer l'élément de pénétration (12) à l'écart du coulage de béton (11),
   au moins un élément de couplage (30) fixé à la structure de support et de guidage (24) et configuré pour se déplacer entre deux positions de fonctionnement, une première position de fonctionnement dans laquelle ledit au moins un élément de couplage (30) est engagé avec et retient ledit au moins un élément de préhension (28), et une seconde position de fonctionnement dans laquelle ledit au moins un élément de couplage (30) est désengagé dudit au moins un élément de préhension (28), et
   un second moyen d'actionnement (32) agencé pour actionner ledit au moins un élément de couplage (30) de manière à ce que ledit au moins un élément de couplage (30) puisse atteindre la première position de fonctionnement ou la seconde position de fonctionnement.

3. Un appareil de mesure (10) selon la revendication 1 ou 2, dans lequel l'élément de pénétration (12) présente une partie d'extrémité proximale (16), opposée à la partie d'extrémité distale (14), ayant une tête proximale (16a) s'étendant radialement d'une plus grande quantité que la partie d'extrémité distale (14).

4. Un ensemble d'usinage capteur (40) pour une voûte et des épaules d'un tunnel (42) en construction définissant une direction axiale (y), comprenant :

   au moins un appareil de mesure (10) selon la revendication 1, 2 ou 3, dans lequel le coffrage comprend également une paroi de construction (44) s'étendant le long de la direction axiale (y) et apte à former un segment de l'intrados de la voûte et des épaules du tunnel (42), et
   dans lequel la paroi frontale (20) est adjacente à la paroi de construction (44) et forme avec elle un coffrage agencé pour contenir un coulage de béton (11),
   un bâti (46) mobile le long de la direction axiale (y) et présentant un intrados (48) et un extrados (50), ledit bâti mobile (46) étant couplé à la paroi de construction (44) et à la paroi frontale (20) et comprenant un troisième moyen d'actionnement (52) agencé sur l'intrados (48) et configuré pour extraire la paroi de construction (44) le long de la direction axiale (y) et un quatrième moyen d'actionnement (54) agencé sur l'extrados (50) et configuré pour extraire la paroi frontale (20) le long de la direction axiale (y).

5. Un ensemble d'usinage capteur (40) selon la revendication 4, dans lequel le premier moyen d'actionnement (18) dudit au moins un appareil de mesure (10) est agencé sur l'extrados (50) du bâti mobile (46) interposé entre le quatrième moyen d'actionnement (54).

6. Un procédé de mesure de la consistance d'un coulage de béton (11) à l'intérieur d'un coffrage comprenant les étapes consistant à :

   a) disposer une paroi de construction (44) s'étendant le long d'une direction axiale (y) et disposer au moins un appareil de mesure (10) selon l'une quelconque des revendications 1 à 3 de manière que la paroi frontale (20) soit adjacente à la paroi de construction (44) et forme avec elle un coffrage ;
   b) disposer un coulage de béton (11) à l'intérieur du coffrage de manière que la surface distale (14a) de l'élément de pénétration (12) entre en contact avec le béton à l'intérieur du coffrage ;
   c) actionner le premier moyen d'actionnement (18) de l'appareil de mesure (10) afin de déplacer l'élément de pénétration (12) vers l'avant en direction du coulage de béton (11) d'une quantité prédéterminée de sorte que la surface distale (14a) de l'élément de pénétration (12) pénètre dans le coulage de béton (11) à l'intérieur du coffrage ;
d) par le biais du moyen de détection (26) de l'appareil de mesure (10) mesurer un signal indicatif d'une charge axiale agissant sur l'interface entre la surface distale (14a) de l'élément de pénétration (12) et le coulage de béton (11) pénétré ; et
e) calculer une valeur de crête (Fn) du signal indicatif de la charge axiale agissant sur l'interface.

7. Un procédé de mesure de la consistance d'un coulage de béton (11) à l'intérieur d'un coffrage pour une voûte et des épaules d'un tunnel (42) en construction comprenant les étapes consistant à :

   a) disposer un ensemble d'usinage capteur (40) pour une voûte et des épaules de tunnel (42) selon la revendication 4 ou 5 à l'intérieur d'une voûte et des épaules d'un tunnel (42) en construction ;
   b) disposer un coulage de béton (11) à l'intérieur du coffrage de manière que la surface distale (14a) de l'élément de pénétration (12) entre en contact avec le béton à l'intérieur du coffrage ;
   c) actionner le premier moyen d'actionnement (18) de l'appareil de mesure (10) afin de déplacer l'élément de pénétration (12) vers l'avant en direction du coulage de béton (11) d'une quantité prédéterminée de sorte que la surface distale (14a) de l'élément de pénétration (12) pénètre dans le coulage de béton (11) à l'intérieur du coffrage ;
   d) par le biais du moyen de détection (26) de l'appareil de mesure (10) mesurer un signal indicatif d'une charge axiale agissant sur l'interface entre la surface distale (14a) de l'élément de pénétration (12) et le coulage de béton (11) pénétré ; et
   e) calculer une valeur de crête (Fn) du signal indicatif de la charge axiale agissant sur l'interface.

8. Un procédé selon la revendication 6 ou 7, dans lequel les étapes c), d) et e) sont répétées séquentiellement avec un intervalle de temps ($\Delta Tn$), jusqu'à ce que la valeur de crête (Fn) du signal indicatif de la charge axiale agissant sur l'interface devienne égale ou supérieure à une valeur limite prédéfinie (Flim).

9. Un procédé selon la revendication 8, dans lequel le calcul de l'intervalle de temps ($\Delta Tn$) comprend les

étapes consistant à :

i) calculer la variation de la valeur de crête ($\Delta$Fn) en fonction de la valeur de crête actuelle (Fn) et de la valeur de crête précédente (Fn-1) ; et

j) calculer l'intervalle de temps ($\Delta$Tn) en fonction de l'intervalle de temps précédent ($\Delta$Tn-1), d'un paramètre représentant la variation souhaitée de la valeur de crête (k) et de la variation de la valeur de crête ($\Delta$Fn).

**10.** Un procédé selon la revendication 8 ou 9, dans lequel l'intervalle de temps ($\Delta$Tn) est compris dans la plage entre une valeur minimale (x) d'environ 45 s et une valeur maximale (y) d'environ 300 s.

**11.** Un procédé selon l'une quelconque des revendications 8 à 10, dans lequel lorsque l'intervalle de temps ($\Delta$Tn) correspond à une valeur de temps minimale (x) pour un nombre prédéterminé de cycles consécutifs (z), le procédé comprenant les étapes consistant à :

m) calculer la valeur moyenne ($\Delta$Fm) des variations de la valeur de crête ($\Delta$Fn) pour le nombre prédéterminé de cycles consécutifs (z) ;

n) comparer les variations de la valeur de crête ($\Delta$Fn) de chacun des cycles consécutifs prédéterminés avec la valeur moyenne ($\Delta$Fm) ; et

o) si les valeurs de crête (Fn) de chacun des cycles consécutifs prédéterminés sont comprises dans la plage entre approximativement a*$\Delta$Fm et approximativement b*$\Delta$Fm, fixer l'intervalle de temps ($\Delta$Tn) à une valeur prédéfinie (h).

fig.2

fig.1

fig.3

fig.4

fig.5

fig.6

fig.7

fig.8

EP 4 495 377 B1

fig.10

fig.9

fig.12

fig.11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO IB2023051394 A
- JP S614908 A
- JP H0270899 A
- JP H08135386 A
- AT 320313 B